# EUROPEAN PATENT APPLICATION

(11) **EP 1 333 667 A1**
(43) Date of publication of application: **06.08.2003**
(21) Application number: 01976654.2
(22) Date of filing: 10.10.2001
(51) Int. Cl.: H04N 5/225

(54) **IRIS IMAGING APPARATUS**

(30) Priority: 16.10.2000 JP 2000315254
(71) Applicant: MATSUSHITA ELECTRIC INDUSTRIAL CO., LTD., Kadoma-shi, Osaka 571-0050 (JP)
(72) Inventor: WADA, Jyoji, Yokohama-shi, Kanagawa 245-0003 (JP); IKOMA, Ken, Yokohama-shi, Kanagawa 225-0023 (JP); TAMURA, Kazushige, Yokohama-shi, Kanagawa 224-0006 (JP); NAKAIGAWA, Tomoyoshi, Yokohama-shi, Kanagawa 244-0802 (JP); DOI, Makoto, Yokohama-shi, Kanagawa 226-0011 (JP); OOI, Koji, Yokohama-shi, Kanagawa 226-0025 (JP); OOTSUNA, Yoshinori, Yokohama-shi, Kanagawa 230-0004 (JP); KOGANE, Haruo, Kawasaki-shi, Kanagawa 214-0038 (JP); KAWAZOE, Syuji, Yokohama-shi, Kanagawa 224-8539 (JP); MIYABAYASHI, Kazuhiro, Tsukui-gun, Kanagawa 220-0105 (JP); WAKIYAMA, Koji, Yokohama-shi, Kanagawa 227-0063 (JP)
(74) Representative: Balsters, Robert
(86) International application number: JP0108889
(87) International publication number: WO02033962

(57) **Abstract**

The object of the invention is to provide iris image pickup apparatus capable of acquiring a high-accuracy iris image in a short time, the iris image pickup apparatus using a low-cost configuration comprising one wide angle camera and one telephotographic camera.

According to the invention, a wide angle camera (25) for picking up an image of an object, a telephotographic camera (22) for bringing the iris of an object into focus and picking up an enlarged image of the iris are provided, as well as a range finder (24) for measuring the distance to the object. Or, the iris image pickup apparatus includes a wide angle camera (25) and a telephotographic camera (22), wherein the long sides of a rectangular image pickup element in the wide angle camera (25) are arranged in the same direction as the height of the object, that is in portrait orientation and that the wide angle camera is mounted on a tilt table (20). Or, the telephotographic camera (22) mounted on the tilt table (20) is arranged so that its optical axis crosses the rotation axis of the tilt table (20) in the same plane. This allows high-speed acquisition of an iris position based on an image picked up by the wide angle camera (25).

## Description

### Technical Field

The present invention relates to iris image pickup apparatus for picking up iris images used for personal authentication in a security system, and in particular to iris image pickup apparatus of a low-cost apparatus configuration and preferable for high-speed pick up of iris images.

### Background Art

In a security system, a method is known to perform authentication by using the wave pattern of the iris of an individual. This method of using an iris, unlike a method using fingerprints, has an advantage of completing authentication by way of image pickup from a distance of an iris in a non-contact way. Thus the method is expected to be diffused in future. However, the image of an iris for use in authentication of an individual must be a clear image focused to improve the recognition rate and must be picked up while the person to be authenticated is at rest.

As a conventional iris image pickup apparatus to satisfy the aforementioned restrictions, there is provided for example iris image pickup apparatus using two types of cameras, a wide angle camera and a narrow angle camera, as described in Japanese Patent Publication No. 2000-23946. In this conventional iris image pickup apparatus, the image of the face of an object person is recognized by way of pattern matching from images of the person picked up by a wide angle camera. Next, the position of the right or left eye of the person is determined from the image of the face. Then, a narrow angle camera as a telephotographic camera is oriented to the iris to bring the iris image into focus with high accuracy by using the auto-focus (AF) technology.

To shorten the time required to bring the iris image into focus using a narrow angle camera, it is necessary only to move the focus lens of the telephotographic camera to a preset position near the focal point at a high speed then start searching for the focal point from the preset position. Thus, in a conventional iris image pickup apparatus, two wide angle cameras are provided and the two wide angle cameras are arranged side by side with a narrow angle camera positioned in the center. This calculates the distance to an object (the preset position) with high accuracy by way of the stereophonic system.

To calculates the iris position from the aforementioned iris image picked up by a wide angle camera, an image picked up by a wide angle camera must contain an image of the face of an object person. In case, as shown in Fig. 11, the head of an object person 1 appears only partially in a picked up image 2 when an eye of an object person 1 is in the picked up image 2, pattern recognition of the position of the "face" is impossible and the iris position cannot be obtained. In case a wide angle camera is mounted on a fixed table as in conventional iris image pickup apparatus, the wide angle camera must be positioned to cover the full image of the face of an object ranging from a tall person to a short person such as a child in an image picked up by the wide angle camera. This results in a considerably small image of the person in the image picked up by the wide angle camera.

In addition, in case only a single wide angle camera is arranged in order to reduce costs, failure to calculates the distance to the iris position increases the focusing search distance by the auto-focus (AF) technology. This may result in the object moving before the iris of the object is brought into focus.

The present invention aims at providing low-cost iris image pickup apparatus for bringing the iris of an object into focus by using a narrow angle camera even in case only a single wide angle camera is used together with the narrow angle camera.

### Disclosure of the Invention

The aforementioned object is attained by iris image pickup apparatus including a wide angle camera for picking up an image of an object, a range finder for measuring the distance to the object, and a telephotographic camera for bringing the iris of the object into focus and picking up an enlarged image of the iris of the object based on the image picked up by the wide angle camera and the measured value of the range finder.

It is impossible to obtain the accurate distance to an object using an image picked up by a single wide angle camera. This problem is solved by using a range finder, which may be a simple one, for determining the distance to the object. Quickly bringing the focal point of a telephotographic camera to the value measured by the range finder and using the auto-focus (AF) technology to search for the focal point considerably reduces the time required to search for the focal point, compared with a case where no distance information is available. By adjusting the position where the telephotographic camera is swung based on the value measured by the range finder, it is possible to arrange the iris image in the center (where the lens aberration is smallest) of the picked up image.

The aforementioned object is also attained by an iris image pickup apparatus including a wide angle camera for picking up an image of an object and a telephotographic camera for bringing the iris of the object into focus and picking up an enlarged image of the iris of the object, characterized in that the long sides of a rectangular image pickup element in the wide angle camera are arranged in the same direction as the height of the object and that the wide angle camera is mounted on a tilt table.

In this way, by arranging the image pickup element of a wide angle camera in portrait orientation, the probability that an image picked up by the wide angle camera contains a full image of the head of the object becomes higher. This reduces the need to re-pick up the image using a wide angle camera thus allowing high-speed acquisition of an iris image. Mounting a wide angle camera on a tilt table allows adjustment of the wide angle camera so that an image picked up by a wide angle camera contains the full image of the head of an object. This provides a full image of the object in the image picked up by the wide angle camera. Thus, the calculation accuracy of the iris position obtained from this picked up image is high. By adjusting the orientation (pan position or tilt position) of the telephotographic camera based on the calculated iris position, it is possible to acquire a high-accuracy iris image at a high speed.

The aforementioned object is also attained by an iris image pickup apparatus including a wide angle camera for picking up an image of an object and a telephotographic camera for bringing the iris of the object into focus and picking up an enlarged image of the iris of the object, wherein on a tilt table are mounted the telephotographic camera whose optical axis is arranged coaxially with the rotation axis of the tilt table, a mirror for panning arranged in front of the telephotographic camera for reflecting the light traveling from the iris and causing the light to be entered in the telephotographic camera, and the wide angle camera arranged so that its optical axis crosses the rotation axis.

Placing the optical axis of the wide angle camera coaxially with the optical axis of the telephotographic camera theoretically eliminates a parallax between the cameras. In practice, such an operation is difficult and some degree of parallax remains. The parallax between the cameras results in a longer time to calculate the iris position to which the telephotographic camera is to be oriented with respect to the iris position obtained from an image picked up by the wide angle camera. Arranging the rotation axis of the tilt table, optical axis of the telephotographic camera and optical axis of the wide angle camera as mentioned earlier eliminates a parallax in the vertical direction, leaving alone a parallax in the horizontal direction (direction of rotation axis of the tilt table). This allows the iris position data obtained from an image picked up by the wide angle camera to be easily converted to coordinate data of the telephotographic camera, thereby reducing the time from image pick up by the wide angle camera to iris pick up by the telephotographic camera.

Preferably, the long sides of a rectangular image pickup element in the wide angle camera are arranged in the same direction as the height of the object and the short sides of the rectangular image pickup element in the telephotographic camera are arranged in the same direction as the height of the object.

By arranging the image pickup element of a wide angle camera in portrait orientation, the probability that an image picked up by the wide angle camera contains a full image of the head of the object becomes higher. By arranging the image pickup element of a telephotographic camera in landscape orientation, the picked up image is extended in the direction a parallax between these cameras is generated. This increases the probability that the iris image is within the picked up image.

Preferably, one axis of the tilt table is directly connected to the output shaft of a tilt motor so that the tilt table may be tilt-operated by the tilt motor, and a damping member is attached to the other axis of the tilt table, the damping member pressed by a spring force against the other axis.

The tilt table is driven to a target position by using a tilt motor such as a stepping motor, the tilt table is halted, then an enlarged image of the iris is picked up by the telephotographic camera. The tilt table is vibrating just after the tilt table is halted, so that it is necessary to wait for the vibration to cease before using the telephotographic camera for picking up. Providing a damping member as in this embodiment shortens the time from the halt of the tilt table until the vibration ceases, thus allowing an iris image to be acquired in a short time.

Preferably, a stop member is provided for pressing the damping member against the other axis to lock the axis while the iris image pickup apparatus is under transportation. Providing a stop member prevents damage to components mounted on a tilt table caused by uncontrollable rotation of the tilt table during transportation of the iris image pickup apparatus.

### Brief Description of the Drawings

Fig. 1 is a front view of an iris image pickup apparatus according to an embodiment of the invention with the outer panel removed.
Fig. 2 is a perspective view of the iris image pickup apparatus shown in Fig. 1.
Fig. 3 is a sectional view taken along the line III-III of Fig. 1.
Fig. 4 is a sectional view taken along the line IV-IV of Fig. 3.
Fig. 5 shows vibrating state of a tilt table assumed when the tilt table is under deceleration at a low speed (a), sudden braking (b) or damped braking (c).
Fig. 6 is a circuit diagram of the electric system of the iris image pickup apparatus shown in Fig. 1.
Fig. 7 is a flowchart showing the operating procedure for the iris image pickup apparatus shown in Fig. 1

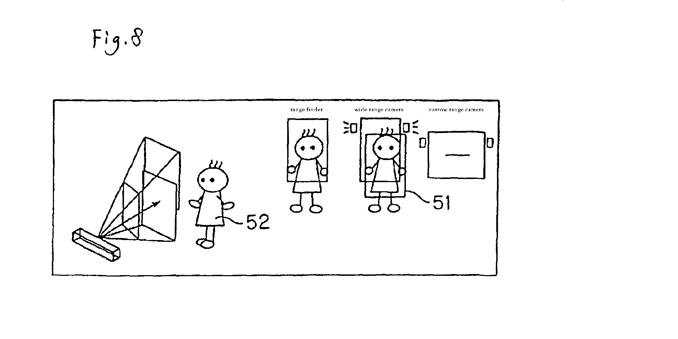

Fig. 8 shows the state of pick up by a wide angle camera.
Fig. 9 is shows an influence of a parallax in the horizontal direction between a wide angle camera and a telephotographic camera.

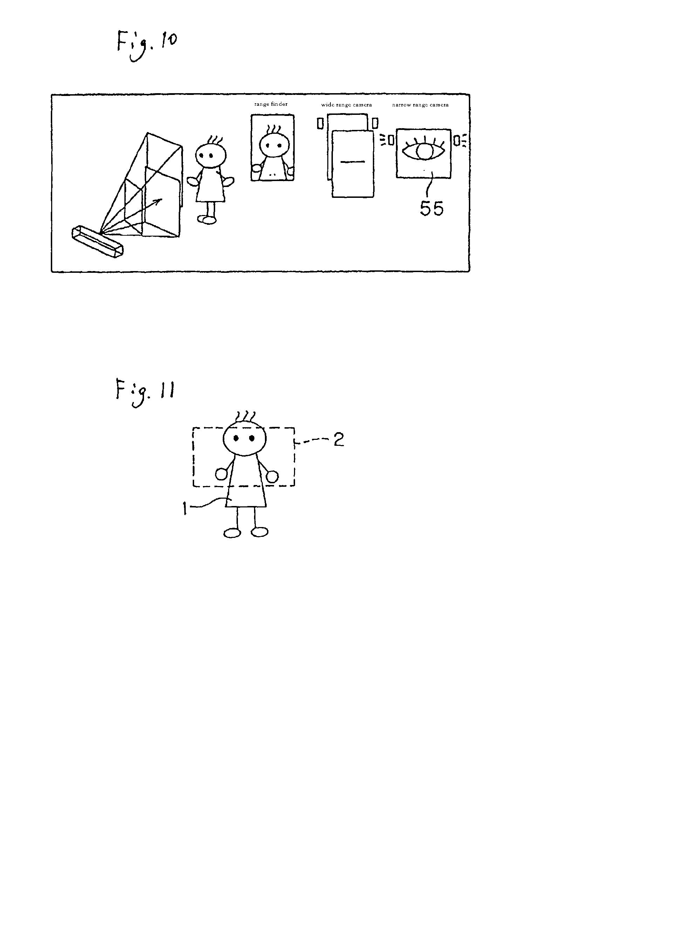

Fig. 10 shows the state of pick up by a telephotographic camera.
Fig. 11 shows an image picked up by a wide angle camera containing partial view of the face of an object.

In the figures, a numeral 10 represents iris image pickup apparatus, 12, 13 an iris lighting fixtures with pan/tilt feature, 14, 15 lighting fixtures for wide angle cameras, 16, 17 support plates, 20 a tilt table, 21 a tilt motor, 22 a telephotographic camera (narrow angle camera) for picking up an iris, 23 a mirror for panning, 24 a range finder, 25 a wide angle camera, 26 panning motor, 20a, pivoting axes of the tilt table, 30 a controller, and 40 a damper.

### Best Mode for Carrying Out the Invention

Iris image pickup apparatus according to the invention will be described referring to the drawings.

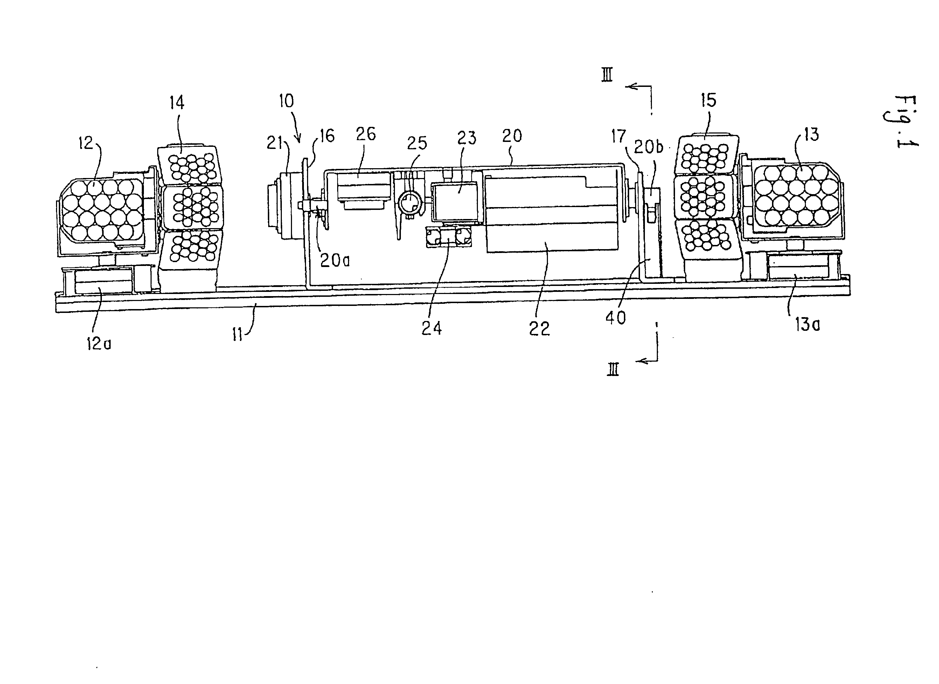

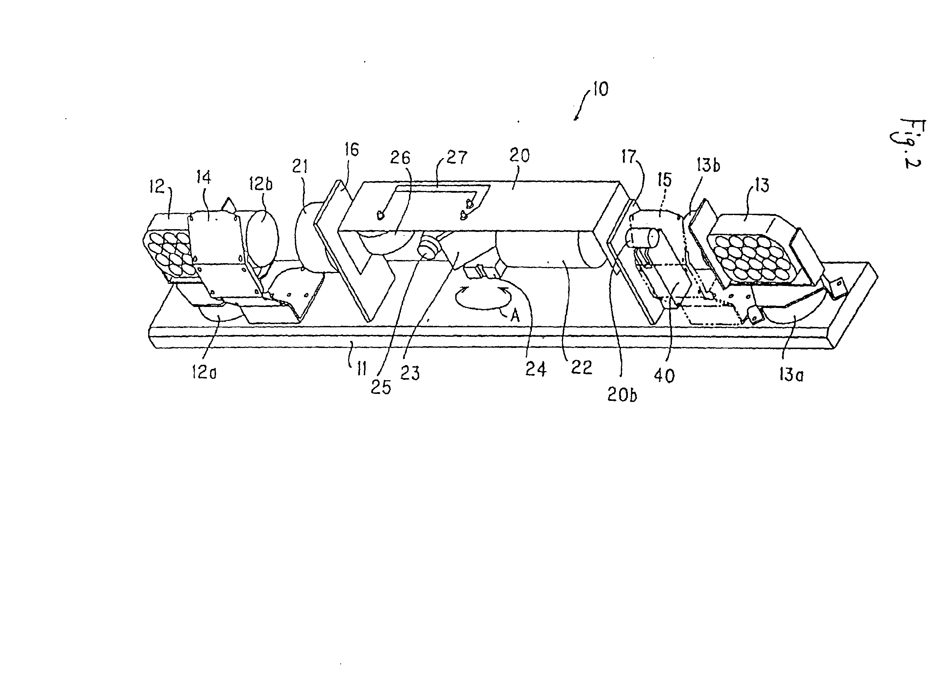

Fig. 1 is a front view of an iris image pickup apparatus according to an embodiment of the invention. Fig. 2 is a perspective view thereof. In both figures, an externally provided panel is not shown.

An iris image pickup apparatus 10 according to this embodiment includes a longitudinal fixed table 11, each of the left and right ends of which are attached iris lighting fixtures 12, 13. Each iris lighting fixture 12, 13 includes a condensing lens for condensing and irradiating infrared rays on the iris and pan motors for lighting 12a, 13a and tilt motors 12b, 13b for lighting in order to orient the illuminating light in the direction of the iris.

Lighting fixtures for wide angle cameras 14, 15 are attached in the inner direction of the fixed table seen from each iris lighting fixtures (in the center direction of the fixed table 11). The lighting fixtures for wide angle cameras 14, 15 have a great number of light emitting diodes. Those light emitting diodes are not shown and only the mounting plate for attaching the light emitting diodes is shown in the perspective view of Fig. 2. The lighting fixtures for wide angle cameras 14, 15 are secured to a fixed table 11 in order to evenly illuminate a wide range with infrared rays (no pan or tilt operation is necessary), with no condensing lenses attached.

A support plate 16 is erected on the fixed table 11 in the inner direction of the fixed table seen from the lighting fixture for wide angle cameras 14 (in the center direction of the fixed table 11). A support plate 17 is erected on the fixed table 11 in the inner direction of the fixed table seen from the lighting fixture for wide angle cameras 15 (in the center direction of the fixed table 11). Between both support plates 16, 17 is attached a tilt table 20.

The tilt table 20 includes pivoting axes 20a, 20b on the left and right respectively. Each axis 20a, 20b is respectively supported rotatably on the support plates 16, 17. One axis 20a is directly coupled to the rotation axis of the tilt motor 21. A damper 40 detailed later is attached on the other axis 20b.

The tilt table 20 mounts a telephotographic camera (narrow angle camera) 22, a mirror for panning 23, a range finder 24, a wide angle camera 25, and a panning motor 26. The telephotographic camera 22 is arranged on the support plate 17 of the tilt table 20 so that the optical axis may be coaxial with the rotation axis of the tilt table 20. The mirror for panning 23 is arranged in front of the telephotographic camera 22. A light reflected on the mirror for panning 23 is entered in the telephotographic camera 22. The mirror for panning 23 is rotatable about the axis perpendicular to the optical axis of the telephotographic camera 22, that is, in the direction of a double-headed arrow A in Fig. 2.

The panning motor 26 for driving the mirror for panning 23 in the direction of the arrow A is attached to the tilt table 20 in a side of the support plate 16 and rotates the mirror for panning 23 via the link mechanism 27. The range finder 24 is also interlocked with the mirror for panning 23 and constantly irradiates infrared rays on an object from the front thus allowing high-accuracy distance measurement. The range finder 24 is also driven by the panning motor 26 via the link mechanism 27.

The wide angle camera 25 is arranged between the mirror for panning 23 and the panning motor 26. The optical axis of the wide angle camera 25 is arranged at a position where it crosses the rotation axis of the tilt table 20 in the same plane. This eliminates a vertical parallax between the wide angle camera 25 and the telephotographic camera 22, leaving alone a horizontal parallax. (Here, it is assumed that the direction of the rotation axis of the tilt table 20 is the transverse (horizontal) direction and the direction of height of an object person is the vertical direction). An image pickup element such as a CCD provided in the wide angle camera 25 is rectangular. The image pickup element is arranged in the wide angle camera 25 so that the long sides thereof are in the vertical direction and the short sides in the horizontal direction. This provides an image picked up by the wide angle camera 25 in portrait orientation.

An image pickup element provided in the telephotographic camera 22 is also rectangular. Unlike the wide angle camera 25, the long sides of the image pickup element are arranged in the horizontal direction and the short sides in the vertical direction. This provides an image picked up by the telephotographic camera 22 in landscape orientation, that is, extending in the direction that a parallax between these cameras is generated.

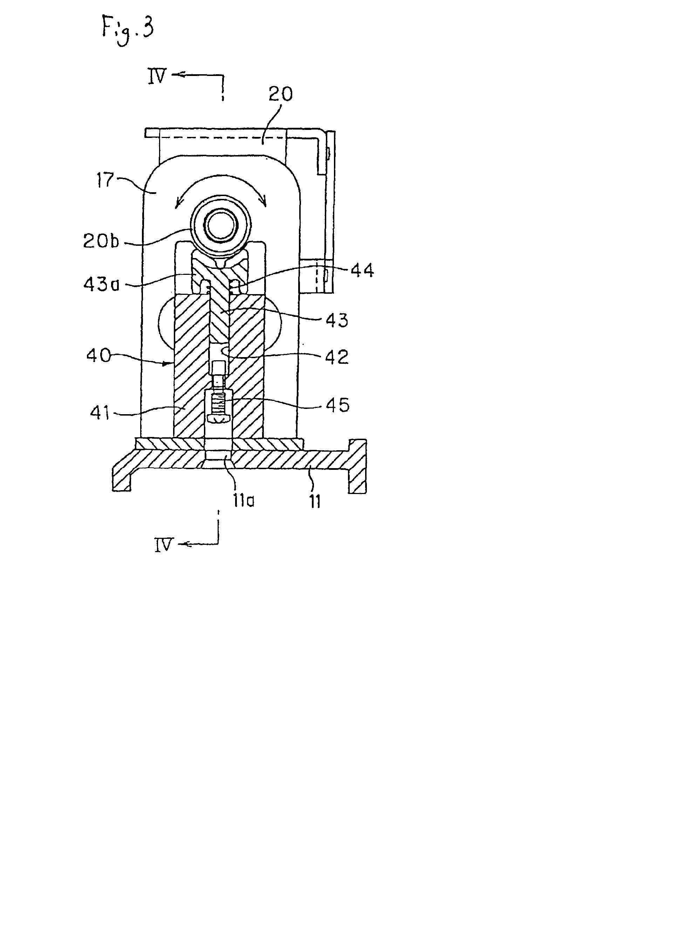

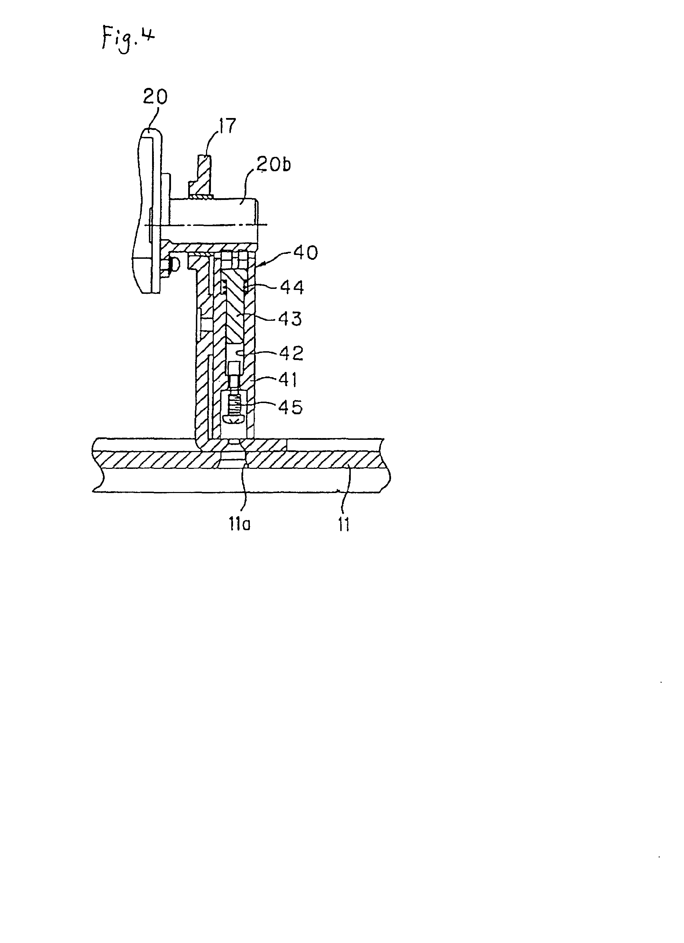

Next, the structure of a damper 40 will be described referring to Figs. 3 and 4. Fig. 3 is a sectional view taken along the line III-III of Fig. 1. Fig. 4 is a sectional view taken along the line IV-IV of Fig. 3. A metallic axis 20b rotatably supported by a support plate 17 penetrates the support plate 17 and projects on the other side of the support plate 17. The damper 40 is provided below the projecting axis 20b.

The damper 40 includes a cylindrical main body 41, a damping member 43 made of resin slidably guided into a central through hole 42 of the main body 41, a resilient spring 44 that presses the U groove of an enlarged head 43a of the damping member 43 against the axis 20b at a predetermined spring pressure, and a screw 45 as a stop member normally secured at a position away from the damping member 43.

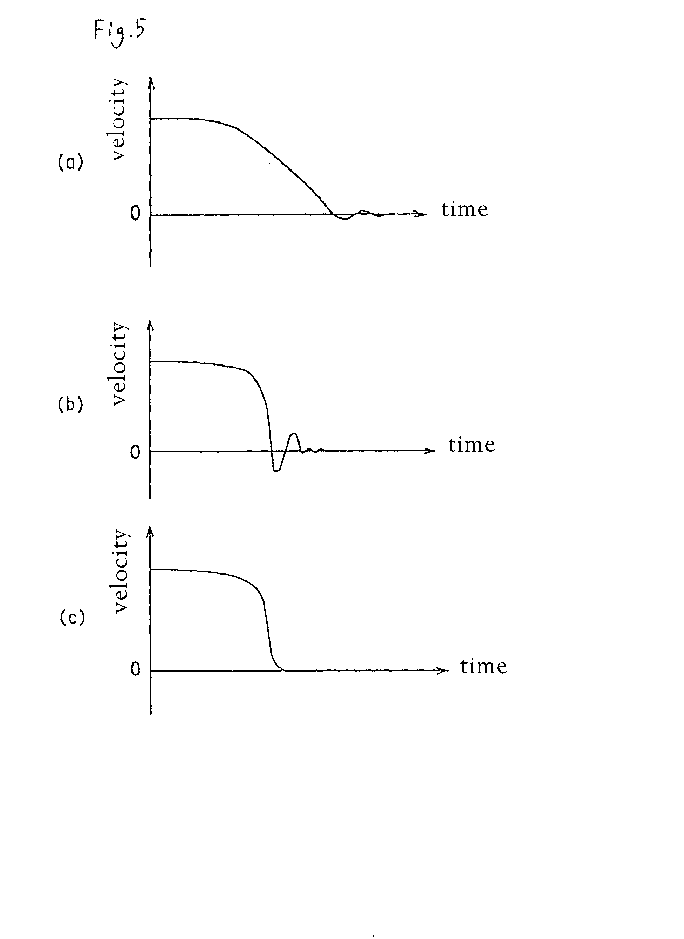

When the iris image pickup apparatus is operated, the tilt table 20 is moved to a predetermined position and halted. In case the tilt table 20 is halted while being decelerated gently, the resulting vibration is not so large as shown in Fig. 5(a). In case the tilt table 20 is moved at a high speed and halted with sudden braking as in Fig. 5(b), a considerable vibration of the tilt table is observed. Such a large vibration in picking up the iris of an object by using a telephotographic camera produces a blurred picked up image . Thus it is necessary to wait until the vibration ceases. This ruins the merit of high-speed operation of the tilt table 20 for quick acquisition of an iris image.

Thus, in this embodiment, a vibration following sudden braking is suppressed by constantly pressing the U groove (groove tailored to the peripheral shape of the axis 20b) of the enlarged head 43a of the damping member 43 against the axis 20b by way of the resilient force of the resilient spring 44, as shown in Fig. 5(c). This allows high-speed operation of the tilt table 20. Because the periphery of the axis 20b is used for braking, the outer diameter of the axis 20b is made larger than that of the axis 20a.

The damper 40 may also be used to lock the tilt table 20. In case the tilt table 20 is rotated during transportation, precious components mounted on the tilt table 20 such as a camera could be damaged. To prevent this, a operator inserts a screwdriver from a through hole 11a provided on the bottom of a fixed table 11 to secure a screw 45 in the direction of the damping member 43 thus pressing the damping member 43 against the axis 20b. This locks the axis 20b and locks the tilt table 20 against free rotation of the tile table 20. The operator loosens the screw 45 to place the screw 45 away from the damping member 43 when installing the iris image pickup apparatus in a predetermined location, the pressing force of the damping member 43 against the axis 43 is a spring force alone, thus allowing rotation of the tilt table 20.

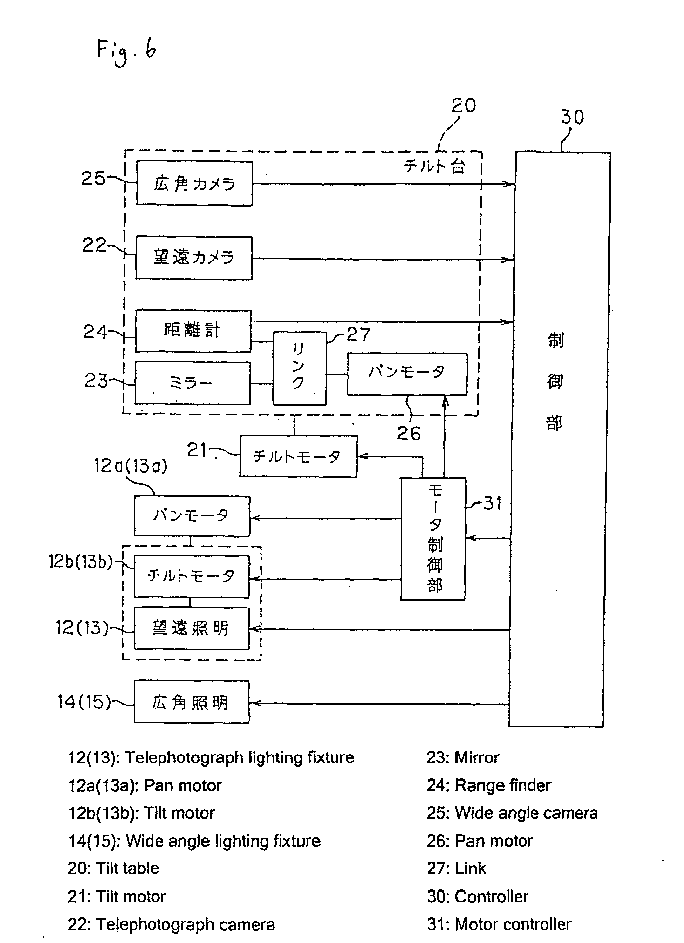

Fig. 6 is a circuit diagram of the electric system of the iris image pickup apparatus shown in Fig. 1. An image (electronic image) picked up by the wide angle camera 25 mounted on the tilt table 20, an image (electronic image) picked up by the telephotographic camera 22 and a value measured by the range finder 24 mounted on the same are inputs of a controller 30 not shown in Fig. 1, and the controller 3 controls the driving of a panning motor 26 and a tilt motor via a motor controller 31, as well as controls driving of a pan motor for lighting 12a (13a) and a tiltmotor for lighting 12b (13b). The controller 30 also controls lighting and shutting off of an iris lighting fixture 12 (13) and a lighting fixture for wide angle cameras 14 (15).

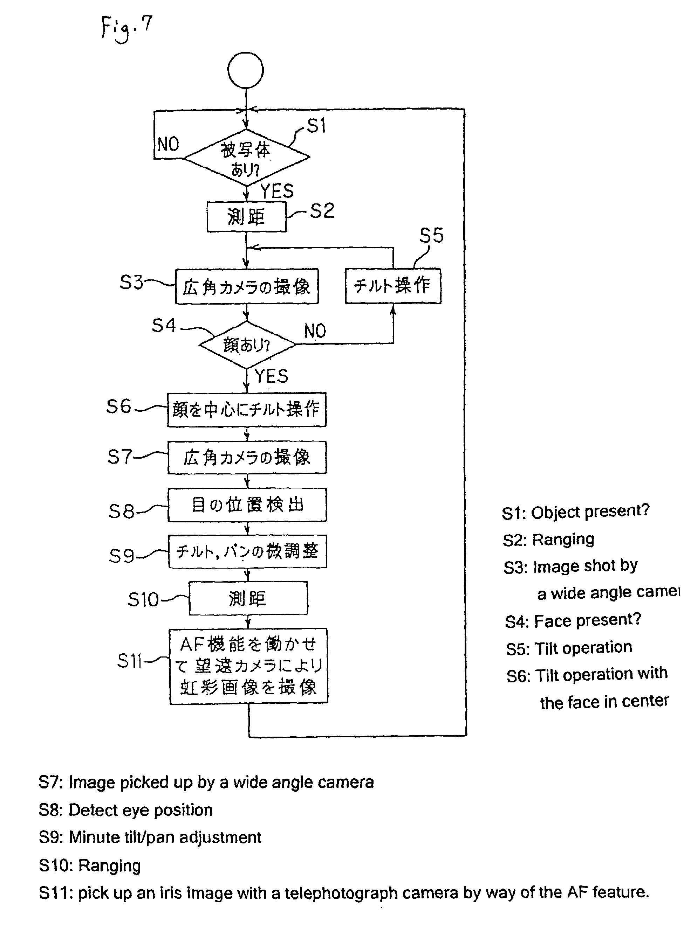

Operation of the iris image pickup apparatus of the foregoing configuration will be described. Fig. 7 is a flowchart showing the operating procedure for the iris image pickup apparatus. In step S1, the iris image pickup apparatus is in the standby state waiting for an object (person to be authenticated) to enter a predetermined front range of the iris image pickup apparatus. In this standby state, each motor 21, 26, 12a, 13a, 12b, 13b is respectively at default position (home position) and the range finder 24 faces the front as a default position also. The range finder 24 emits infrared rays in the standby state constantly or per predetermined time. The presence/absence of a resulting reflected light allows the controller 30 shown in Fig. 6 to determine the presence of an object. In case the object has entered the image pickup range, the distance to the object is measured by the range finder based on the reflected light (step S2) and execution proceeds to step S3. In case the iris image pickup apparatus is applied to an ATM terminal of a bank, the standby step of waiting for an object may be replaced by a step of waiting for a customer to start ATM operation.

In step S3, images are picked up by the wide angle camera 25. At this time, lighting fixtures for wide angle cameras 14, 15 are illuminated. In step S4, the controller 30 uses pattern matching to determine whether the face of the object is in the image picked up. In case the face of the object is not found, the controller 30 outputs a driving instruction to a tilt motor 21 via a motor controller 31 to per form tilt operation (step S5), then captures the image picked up by the wide angle camera in step S3. The subsequent steps S3, S4, S5 are repeated until the whole face image is captured.

The image pickup element of the wide angle camera 25 is in portrait orientation so that the probability that an image picked up by the wide angle camera contains the face of an object 52 becomes higher. Tilt operation allows swinging of the optical axis of the telephotographic camera in vertical direction so that it is easy to capture an image of the face of the object.

In case the face pattern is found in the image picked up by the wide angle camera, execution proceeds from step S4 to step S6. Tilt operation of the tilt table 20 is made so that the face comes in the center of the image picked up by the wide angle camera. Pan position and tilt position of the iris lighting fixtures 12, 13 in Fig. 1 are adjusted. The illuminating light irradiation direction is swiveled in advance so that the light illuminates the face. The mirror for panning 23 is swiveled in advance so that the telephotographic camera 22 can pick up the face. Corresponding accurate adjustment is made in step S9 as mentioned later. In step S7, an image is picked up by the wide angle camera. Execution then proceeds to step S8.

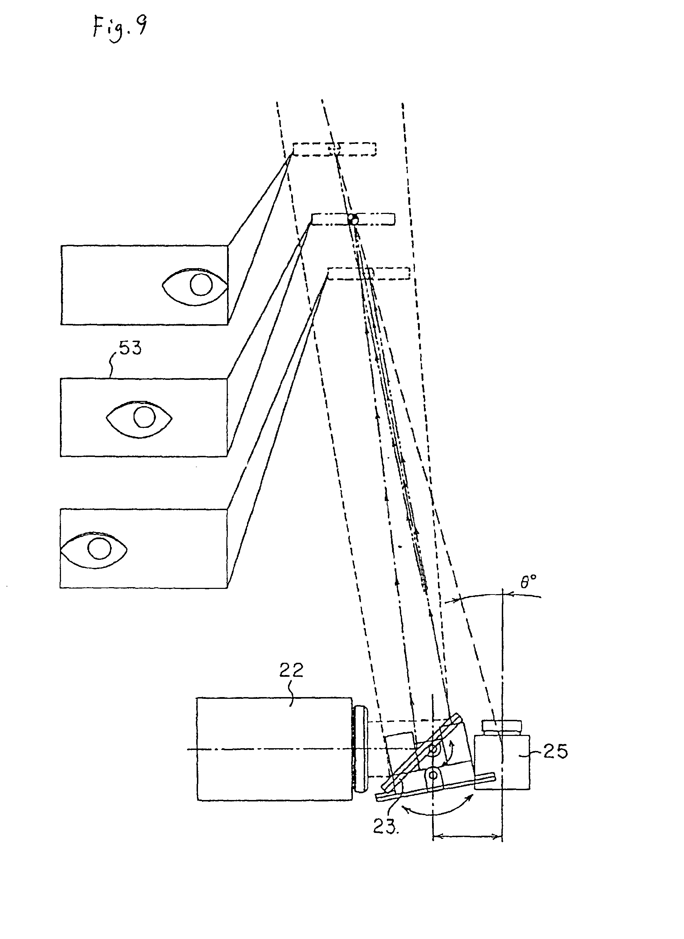

In step S8, the controller 30 detects the position of the left eye or right eye from the image picked up by the wide angle camera captured in step S7. From the image picked up by the wide angle camera, the direction an eye is present is known, but the distance to the "eye" picked up is unknown. When the distance is unknown, a parallax in the right/left direction is present between the wide angle camera 25 and the telephotographic camera 22. Thus the position of the iris image in the screen is dislocated depending on the swivel angle of the mirror for panning 23. Because an aberration is present in a camera lens, an iris image is preferably in the center of a picked up image to obtain an iris image with high authentication accuracy (image 53 in Fig. 9). Thus, the controller 30 also uses the measurement value of the range finder measured in step S2 to calculate the position of an eye to be picked up.

Next, the controller 30 converts the position of an eye obtained in step S8 to coordinates of the telephotographic camera 22. The controller 30 then obtains the accurate tilt position of the tilt table 20 and the accurate pan position of the mirror for panning 23 as well as the accurate tilt position and pan position of the iris lighting fixtures 12, 13, to carry out minute adjustment of the tilt position and the pan position (step S9), so that the telephotographic camera 22 will capture the iris with high accuracy and so that the focused illuminating light of the iris lighting fixture 12, 13 will be irradiated on the iris.

In step S10, the controller 30 uses the range finder 24 to measure the distance to the iris. The range finder 24 is interlocked with the pan position adjustment of the mirror for panning 23 and its range direction is adjusted accordingly. Thus the infrared rays for measurement are irradiated toward the iris. Receiving a light reflected on a cheek below an eye for example allows accurate measurement of the distance to the iris.

In the next step S11, the distance to the iris measured in step S10 is preset to the telephotographic camera 22, a focus lens (not shown) is rapidly driven to focus on the distance. Subsequently, a general auto-focus (AF) technology is used to bring the iris in focus. For example, a focus lens driving motor is traveled step by step to obtain the image 55 picked up by the telephotographic camera 22. A focus lens position where the high-frequency component in the image is highest is assumed as a focus. The image then obtained is acquired as an iris image to be authenticated. When the telephotographic camera 22 is used to pick up an iris image, the controller 30 illuminates either or both of the iris lighting fixtures 12, 13. The controller 30 then passes the iris image picked up at the focus to authentication apparatus (not shown). Execution returns to step 1.

As mentioned earlier, this embodiment uses a single wide angle camera and a range finder is used to measure the distance to an object. This simplifies the apparatus configuration and reduces manufacturing costs. The focal point of a telephotographic camera is quickly brought to the value as a distance to the object measured by the range finder which may be a simple one, and the auto-focus (AF) technology is subsequently used to search for the focal point. The direction the telephotographic camera is adjusted is determined based on the measured value of the range finder to include an iris image in the center of a picked up image. This approach obtains a highly accurate iris image in a short time.

In this embodiment, the image pickup element of a wide angle camera is arranged in portrait orientation so that the probability that an image picked up by the wide angle camera contains a full image of the head of the object becomes higher. This reduces the need to re-pick up the image using a wide angle camera thus allowing high-speed acquisition of an iris image. Mounting a wide angle camera on a tilt table allows adjustment of the wide angle camera so that an image picked up by a wide angle camera contains the full image of the head of an object. This provides a full image of the object in the image picked up by the wide angle camera. Thus, the calculation accuracy of the iris position obtained from this picked up image is high. By adjusting the orientation (pan position or tilt position) of the telephotographic camera based on the calculated iris position, it is possible to acquire a high-accuracy iris image at a high speed.

According to the embodiment, the optical axis of the wide angle camera is arranged at a position where it crosses the rotation axis of the tilt table in the same plane. This eliminates a vertical parallax between the wide angle camera and the telephotographic camera, leaving alone a horizontal parallax. This allows the iris position data obtained from an image picked up by the wide angle camera to be converted to coordinate data of the telephotographic camera with ease, that is in a short calculation time, thereby reducing the time from image pick up by the wide angle camera to iris pick up by the telephotographic camera.

According to the embodiment, a mechanical braking mechanism (damper 40) is provided against rotation of the tile table thus suppressing any vibration. This allows the tile table to be driven at a high speed and suddenly braked thereby further reducing the time to acquisition of an iris image.

While the invention has been described in detail referring to a specific embodiment, those skilled in the art will appreciate that the invention may be modified or corrected in various forms without departing from the spirit and the range of the invention.

This application is based on the Japanese Patent Application No. 2000-315254 filed October 16, 2000, which is incorporated herein by reference.

### Industrial Applicability

According to this invention, iris image pickup apparatus is provided capable of acquiring a high-accuracy iris image in a short time, the iris image pickup apparatus using a low-cost configuration comprising one wide angle camera and one telephotographic camera.

## Claims

1. An iris image pickup apparatus comprising:
a wide angle camera for picking up an image of an object;
a range finder for measuring the distance to said object; and
a telephotographic camera for bringing the iris of said object into focus and picking up an enlarged image of the iris of said object based on the image picked up by said wide angle camera and the measured value of said range finder.

2. An iris image pickup apparatus comprising:
a wide angle camera for picking up an image of an object; and
a telephotographic camera for bringing the iris of said object into focus and picking up an enlarged image of the iris of said object,
wherein the long sides of a rectangular image pickup element in said wide angle camera are arranged in the same direction as the height of said object,
wherein the wide angle camera is mounted on a tilt table.

3. An iris image pickup apparatus comprising:
a wide angle camera for picking up an image of an object; and
a telephotographic camera for bringing the iris of said object into focus and picking up an enlarged image of the iris of said object,
wherein said telephotographic camera whose optical axis is arranged coaxially with the rotation axis of a tilt table, a mirror for panning arranged in front of the telephotographic camera for reflecting the light traveling from said iris and allowing the light to be entered in said telephotographic camera, and said wide angle camera arranged so that its optical axis crosses said rotation axis in the same plane are mounted on said tilt table.

4. The iris image pickup apparatus according to claim 3,
wherein the long sides of a rectangular image pickup element in said wide angle camera are arranged in the same direction as the height of said object,
wherein the short sides of the rectangular image pickup element in said telephotographic camera are arranged in the same direction as the height of said object.

5. The iris image pickup apparatus according to claim 3,
wherein one axis of said tilt table is directly connected to the output shaft of a tilt motor so that the tilt table may be tilt-operated by said tilt motor,
wherein a damping member is attached to the other axis of said tilt table, said damping member pressed by a spring force against the other axis.

6. The iris image pickup apparatus according to claim 5, wherein a stop member is provided for pressing said damping member against said other axis to lock the axis while the iris image pickup apparatus is under transportation.

7. A damper provided on apparatus having a member rotating about an axis comprising:
a damping member provided in contact with said axis;
a stop member for pressing the damping member against said axis to lock the axis; and
a spring member for pressing said damping member against said axis at a spring pressure when the stop member is placed away from said damping member.
